# EUROPÄISCHE PATENTSCHRIFT

(11) **EP 4 588 882 B1**
(45) Veröffentlichungstag und Bekanntmachung des Hinweises auf die Patenterteilung: **09.09.2026**
(21) Anmeldenummer: 25152163.9
(22) Anmeldetag: 16.01.2025
(51) Int. Cl.: B67C 3/00, A61L 2/208, B29C 49/42, A61L 2/16, A61L 2/186, B29C 49/46

(54) **BEHÄLTERBEHANDLUNGSANLAGE SOWIE VERFAHREN ZUM BETRIEB**
CONTAINER TREATMENT SYSTEM AND METHOD FOR OPERATING
INSTALLATION DE TRAITEMENT DE RÉCIPIENTS ET PROCÉDÉ DE FONCTIONNEMENT

(30) Priorität: 18.01.2024 DE 102024101383
(43) Veröffentlichungstag der Anmeldung: 23.07.2025
(73) Patentinhaber: KHS GmbH, 44143 Dortmund (DE)
(72) Erfinder: Gerhards, Martin, 44143 Dortmund (DE); Drenguis, Alfred, 44143 Dortmund (DE)
(74) Vertreter: Andrejewski - Honke Patent- und Rechtsanwälte Partnerschaft mbB

(56) Entgegenhaltungen:
- WO-A1-2009/132686
- WO-A1-2022/112301

## Beschreibung

Die Erfindung betrifft eine Behälterbehandlungsanlage zum Behandeln von Behältern mit zumindest einem Behandlungsmodul, welches eine Behälteraufnahme sowie eine der Behälteraufnahme zugeordnete Fluidzuführung aufweist, wobei die Fluidzuführung dazu ausgebildet ist, ein Behandlungsfluid in einen Behälter einzubringen und wobei die Fluidzuführung fluidwirkend an eine Zuführleitung anschließt.

Die Erfindung bezieht sich insbesondere auf Behälterbehandlungsanlagen aus dem Bereich der Lebensmittelindustrie, insbesondere der Getränkeindustrie, wobei mit einer Behandlung sämtliche Tätigkeiten gemeint sind, welche die Behälter in irgendeiner Form beeinflussen, sofern ein hierzu Behandlungsfluid für die Behandlung verwendet wird.

Beispielsweise kann es sich bei der Behälterbehandlungsanlage um eine Füllanlage handeln, in welcher die Behälter mit einem Füllgut befüllt werden. Hierbei kann es sich beispielsweise auch um ein flüssiges Lebensmittel, insbesondere ein Getränk, handeln, welches in der Behälterbehandlungsanlage in die Behälter eingefüllt wird. In diesem Fall handelt es sich bei den Behältern insbesondere um Getränkeflaschen z.B. aus Glas oder aus Kunststoff oder um Getränkedosen.

Alternativ kann die Behälterbehandlungsanlage auch als Umformanlage ausgebildet sein, in welcher die Behälter zunächst zu Getränkeflaschen umgeformt werden. Dies erfolgt üblicherweise in Blasanlagen oder Streckblasanlagen, wobei die Behälter zunächst als sogenannte Vorformlinge bzw. Preforms vorliegen.

Unter Vorformlingen oder Preforms werden Behälter verstanden, welche lediglich im Bereich der Behältermündung fertig mit einem Außengewinde zur Aufnahme einer Verschlusskappe ausgebildet sind und wobei dann lediglich der Behälterkörper im Rahmen der Blassumformung oder der Streckblasumformung zu einer Getränkeflasche ausgebildet wird. Hierzu wird ein Behandlungsfluid, insbesondere ein gasförmiges Behandlungsfluid (z.B. Druckluft), in die Behälter eingebracht, welches diese mit einem Innendruck beaufschlagt und den Behälterkörper an eine Blaskavität herangedrückt. Die Blaskavität weist die Form der zu fertigenden Getränkeflasche auf. Bei einer Streckblasanlage ist darüber hinaus eine sogenannte Reckstange vorgesehen, welche eine axiale Längung des Behälterkörpers entlang der Behälterachse bewirkt.

Um eine solche Blasumformung zu ermöglichen, sind die Behälter üblicherweise aus einem thermoplastischen Material gebildet, welches zunächst innerhalb einer Heizvorrichtung erwärmt wird und im Zuge dessen aufweicht, Bei dem Material handelt es sich insbesondere um Polyethylenterephthalat (PET).

Im Rahmen der Blasumformung oder der Streckblasumformung kann auch ein flüssiges Behandlungsfluid in die Behälter eingebracht werden. Hierbei handelt es sich dann vornehmlich um ein Füllgut, welches ohnehin zur Befüllung der Behälter vorgesehen ist. Entsprechend werden die Behälter nicht nur durch Einbringen des Behandlungsfluids umgeformt sondern zugleich auch befüllt. Ein solches Verfahren wird gemeinhin auch als Formfill-Verfahren bezeichnet.

Hierbei ist es unabhängig von der Ausgestaltung der Behälterbehandlungsanlage üblich, die Fluidzuführung der Behandlungsmodule und auch die Zuführleitung in gewissen Zeitabständen zu sterilisieren. Unter einem Sterilisieren wird im Rahmen der Erfindung ein Verfahren verstanden, welches die Abtötung von Keimen und Bakterien ermöglicht. Entsprechend erfolgt nicht nur eine einfache Reinigung der Fluidzuführungen, bei der Keime oder Bakterien lediglich herausgewaschen werden. Vielmehr wird aktiv ein Sterilisationsmittel eingebracht, welches dazu in der Lage ist, die vorhandenen Bakterien abzutöten.

Um eine entsprechende Sterilisation durchführen zu können wird unterschieden zwischen einem Produktionsbetrieb und einem Sterilisationsbetrieb, wobei während des Sterilisationsbetriebes keine aktive Behandlung der Behälter erfolgen kann, sodass entsprechend üblicherweise auch keine Behälter innerhalb der Behälterbehandlungsanlage angeordnet sind.

Aus der EP 2 388 126 B1 ist es bekannt, dass die Zuführleitung in einer Blasanlage über eine Anschlussleitung mit einer Sterilisationsvorrichtung verbunden wird, über die dann ein Sterilisationsmittel enthaltendes Trägerfluid in die Zuführleitung und dann über eine Drehdurchführung in die einzelnen Fluidzuführungen eingebracht wird. Nach der Sterilisation kann dann die Zuführleitung in einfacher Weise von der Anschlussleitung getrennt werden, sodass über die Zuführleitung im Zuge der Aufnahme des Produktionsbetriebes erneut ein Blasfluid in die Vorformlinge eingebracht werden kann.

Die WO 2022/112301 A1 offenbart eine Behälterbehandlungsanlage, nämlich eine Blasmaschine, bei der in einem Sterilisationsbetrieb mittels einer Sterilisationsvorrichtung ein Sterilisationsmittel in die Fluidzuführungen mittels eines Anschlusses von einer Sterilisationsmittelquelle eingebracht wird.

Aus der WO 2009/132686 A1 ist eine Vorrichtung zum Bereitstellen eines Sterilisationsmittels in der Verpackungsindustrie bekannt, wobei das Sterilisationsmittel in einer Zerstäuberkammer gemischt und dann einer Behälterbehandlungsanlage zugeführt wird.

Wenngleich sich die aus der Praxis bekannten Lösungen zum Sterilisieren der Fluidleitungen grundsätzlich bewährt haben, muss stets eine separate Sterilisationsvorrichtung bereitgestellt werden, welche dann in einem Sterilisationsbetrieb mit der Zuführleitung verbunden wird. Dies erfordert einerseits einen erhöhten Platzbedarf. Zugleich ist es auch erforderlich, entsprechende Maßnahmen vorzuhalten, welche sicherstellen, dass während des Produktionsbetriebes kein Sterilisationsmittel in das Leitungssystem gelangt.

Vor diesem Hintergrund liegt der Erfindung die Aufgabe zugrunde, eine Behälterbehandlungsanlage anzugeben, welche in einfacher Art und Weise eine Sterilisation der Fluidzuführungen und der Zuführleitung ermöglicht.

Gegenstand und Lösung dieser Aufgabe ist eine Behälterbehandlungsanlage gemäß Patentanspruch 1. Demnach ist erfindungsgemäß vorgesehen, dass in einem Sterilisationsbetrieb zumindest ein Sterilisationsmittelreservoir in der Zuführleitung angeordnet ist, welches fluidwirkend mit der Fluidzuführung des zumindest ein Behandlungsmoduls verbunden ist.

Entsprechend wird ein Sterilisationsmittelreservoir in die Zuführleitung integriert, welches dann während des Sterilisationsbetriebes fluidwirkend innerhalb der Zuführleitung einsetzbar ist, sodass ein in dem Sterilisationsmittelreservoir angeordnetes Sterilisationsmittel aus dem Sterilisationsmittelreservoir ausgetragen und in die Zuführleitung sowie anschließend in die Fluidzuführungen des zumindest einen Behandlungsmoduls eingebracht wird.

Die Behälterbehandlungsanlage ist gemäß einer bevorzugten Ausgestaltung als Blasanlage oder Streckblasanlage ausgebildet, in welcher Behälter in Form von Vorformlingen zu Getränkeflaschen umgeformt werden. Das Umformen erfolgt entweder mit einem gasförmigen Behandlungsfluid (z.B. Druckluft) oder mit einem flüssigen Behandlungsfluid, wobei es sich dann vornehmlich um ein flüssiges Füllgut, insbesondere ein Lebensmittel z.B. ein Getränk, handelt (Formfill-Verfahren). Um eine solche Umformung zu ermöglichen, weisen die Behandlungsmodule jeweils eine als Blasform ausgebildete Behälteraufnahme auf. Diese Blasform besteht üblicherweise aus zumindest zwei Blasformhälften, welche verschwenkbar zueinander ausgebildet sind und welche gemeinsam eine Blaskavität bilden, welche der Form der zu fertigenden Getränkeflasche entspricht.

Im Öffnungszustand der Blaskavität kann ein Behälter in Form eines Vorformlings eingesetzt werden, wobei die Blasform sodann durch Verschwenken der Blasformhälften in den Schließzustand überführt wird. Der Behälteraufnahme ist eine Fluidzuführung zugeordnet, über welche im Schließzustand das Behandlungsfluid unter Druck in die Behälter eingeleitet wird, um eine plastische Verformung des Behälters zu bewirken. Üblicherweise ist die Fluidzuführung an einer oberhalb der Behälteraufnahme angeordneten Ventileinrichtung gebildet, so dass entsprechend die Zufuhr des Behandlungsfluids über die Ventileinrichtung gesteuert werden kann.

Das zumindest eine Behandlungsmodul ist üblicherweise an einem rotierbar antreibbaren Träger angeordnet, so dass eine Behandlung der Behälter im Zuge der Rotation des Trägers erfolgt. Auch bei einer Füllanlage ist eine solche Ausgestaltung bevorzugt. Insbesondere sind eine Vielzahl, z.B. 10 bis 20 Behandlungsmodule, an dem Träger angeordnet, so dass eine Vielzahl von Behälter gleichzeitig behandelt werden können. Gemäß einer solchen Ausgestaltung sind die Behandlungsmodule einschließlich der Fluidzuführungen und der Behälteraufnahme fest auf dem Träger montiert, so dass entsprechend die Zuleitung des Behandlungsfluids in die Behälter in einem rotierenden System erfolgt. Hierzu schließen die Fluidzuführungen entweder unmittelbar oder mittelbar an eine Drehdurchführung an, welche die Übertragung des Behandlungsfluids zwischen einem feststehenden Leitungssystem und den rotierenden Fluidzuführungen ermöglicht. Gemäß einer solchen Ausgestaltung ist die Zuführleitung Teil des feststehenden Leitungssystems und schließt endseitig an die Drehdurchführung an.

Um eine plastische Verformung der Behälter überhaupt erst in geeigneter Art und Weise zu ermöglichen, kann die Behälterbehandlungsanlage eine Heizvorrichtung aufweisen, welche dem zumindest einen Behandlungsmodul vorgeschaltet ist und in welcher die Behälter bzw. die Vorformlinge aufgeheizt werden, um das Material, insbesondere PET, aufzuweichen.

Die Erfindung ist darüber hinaus gerade dann von Vorteil, wenn nicht nur das Einbringen eines sterilen Behandlungsfluids gewünscht wird, sondern wenn auch die Behälter bereits während der Behandlung steril vorliegen oder im Zuge der Behandlung sterilisiert werden. Insbesondere kann die Behälterbehandlungsanlage eine Behältersterilisationsvorrichtung aufweisen, in welcher die Behälter sterilisiert werden. Entsprechend ist zu unterscheiden zwischen einer erfindungsgemäßen Sterilisationsvorrichtung, welche das Leitungssystem, insbesondere die Zuführleitung und die Fluidzuführungen, in einem Sterilisationsbetrieb sterilisiert und einer Behältersterilisationsvorrichtung, welche im Produktionsbetrieb die Behälter sterilisiert.

Bevorzugt ist diese Behältersterilisationsvorrichtung in Transportrichtung der Behälter vor dem zumindest einen Behandlungsmodul angeordnet. Im Falle einer Blasanlage kann die Behältersterilisationsvorrichtung entweder vor der Heizvorrichtung oder zwischen der Heizvorrichtung und dem zumindest einen Behandlungsmodul angeordnet sein. Eine Anordnung vor der Heizvorrichtung weist hierbei den Vorteil auf, dass im Falle des Sterilisierens mit Hilfe eines Behältersterilisationsfluids erst in der Behältersterilisationsvorrichtung das Behältersterilisationsfluids eingebracht und dieses Behältersterilisationsfluids sodann in der nachgeschalteten Heizvorrichtung aktiviert wird. Bei dem Sterilisationsmittel handelt es sich insbesondere um Wasserstoffperoxid (H2O2), Peressigsäure, Wasserdampf oder Mischungen hieraus. Darüber hinaus ist es auch denkbar, dass die Behältersterilisationsvorrichtung in die Heizvorrichtung oder in die Behandlungsmodule integriert ist, so dass eine Sterilisation entsprechend während der Erwärmung oder während der Blasumformung erfolgt. Anstelle einer Sterilisation mit einem Behältersterilisationsfluid kommt ferner auch eine Sterilisation der Behälter durch Bestrahlung, insbesondere durch UV-Bestrahlung, in Betracht.

Das Sterilisationsmittelreservoir weist im Wesentlichen ein Volumen auf, welches ausreichend ist, um die Fluidzuführungen sowie die Zuführleitung in ausreichender Art und Weise während des Sterilisationsbetriebes zu sterilisieren. Insbesondere kann das Volumen zwischen 0,2 und 10 l betragen. Üblicherweise erfolgt der Sterilisationsbetrieb in bestimmten Abständen z. B. einmal pro Woche und wechselt sich mit einem daran anschließenden Produktionsbetrieb ab. Die Größe des Sterilisationsmittelreservoirs ist dann bevorzugt ausreichend für zumindest einen Sterilisationsbetrieb.

Gemäß einer bevorzugten Ausgestaltung der Erfindung ist das zumindest eine Sterilisationsmittelreservoir umströmbar ausgebildet. Entsprechend kann das Sterilisationsmittelreservoir eine oder mehrere Öffnungen z.B. Ventile aufweisen, über die das Sterilisationsmittel in das Trägerfluid geleitet wird. Durch die Umströmung des Sterilisationsmittelreservoirs nimmt das Trägerfluid das Sterilisationsmittel auf und bildet gemeinsam mit diesem ein Sterilisationsfluid. Dieses Sterilisationsfluid aus Sterilisationsmittel und Trägerfluid wird dann durch die Zuführleitung sowie die Fluidzuführungen geführt und durchströmt und sterilisiert im Zuge dessen die einzelnen Fluidzuführungen.

Bei dem in dem Sterilisationsmittelreservoir angeordneten Sterilisationsmittel handelt es sich insbesondere um Wasserstoffperoxid (H₂O₂) oder Peressigsäure, welche üblicherweise zunächst innerhalb des Sterilisationsmittelreservoirs verdampfen und sich sodann mit dem Trägerfluid vermischen. Bei dem Trägerfluid handelt es sich bevorzugt um Luft, insbesondere um trockene Luft, wobei unter trockener Luft im Rahmen der Erfindung Luft verstanden wird, welche einen Feuchtegehalt von weniger als 5 % bei einer Temperatur von 90 °C aufweist.

Eine bevorzugte Weiterbildung der Erfindung sieht vor, dass das zumindest eine Sterilisationsmittelreservoir als austauschbare Sterilisationsmittelpatrone oder als nachfüllbarer Sterilisationsmitteltank in der Zuführleitung angeordnet ist. Wie bereits zuvor erläutert, reicht das in dem Sterilisationsmittelreservoir angeordnete Sterilisationsmittel üblicherweise für einen Sterilisationsbetrieb aus, wobei dann erneut Sterilisationsmittel in dem Sterilisationsmittelreservoir angeordnet werden muss. Dies kann beispielsweise dadurch ermöglicht werden, dass das zumindest ein Sterilisationsmittelreservoir als Sterilisationsmittelpatrone ausgebildet ist, wobei diese Sterilisationsmittelpatrone vollständig austauschbar ausgebildet ist.

Folglich wird die im Wesentlichen entleerte Sterilisationsmittelpatrone nach dem Sterilisationsbetrieb bzw. am Ende des Sterilisationsbetriebes aus der Zuführleitung entnommen und durch eine neue, befüllte Sterilisationsmittelpatrone ersetzt. Das Einsetzen der neuen Sterilisationsmittelpatrone kann auch erst zu Beginn eines erneuten Sterilisationsbetriebes erfolgen, sodass dann während des Produktionsbetriebes keine Sterilisationsmittelpatrone in der Zuführleitung angeordnet ist. Entsprechend weist die Zuführleitung aber entsprechende Aufnahmemittel auf, welche die Festlegung der zumindest einen Sterilisationsmittelpatrone innerhalb der Zuführleitung ermöglichen. Alternativ kann auch gegen Ende des Sterilisationsbetriebes bzw. zu Beginn eines erneuten Sterilisationsbetriebes eine Befüllung eines Sterilisationsmitteltanks erfolgen, sodass dann entsprechend lediglich das Nachfüllen des Sterilisationsmittels erforderlich ist. Wenngleich beide Lösungen praktikable Wege darstellen, so weist der komplette Austausch einer Sterilisationsmittelpatrone den Vorteil auf, dass dieser Vorgang im Hinblick auf die Arbeitssicherheit von besonderem Vorteil ist, da das Sterilisationsmittel während des Austausches vollständig gekapselt in der Sterilisationsmittelpatrone vorliegt. Sofern lediglich ein Befüllen eines Sterilisationsmitteltanks erfolgt, ist sicherzustellen, dass während dessen kein Sterilisationsmittel in die Umgebung gelangt.

Um einen solchen Austausch oder ein Befüllen des Sterilisationsmittelreservoirs zu ermöglichen, ist es erforderlich, die Zuführleitung entsprechend auszubilden. Bevorzugt ist daher die Zuführleitung abschnittsweise als Sterilisationsvorrichtung ausgebildet, wobei dass zumindest eine Sterilisationsmittelreservoir in einem fluidwirkend mit der Zuführleitung verbundenen Kanalabschnitt der Sterilisationsvorrichtung angeordnet ist. Der Kanalabschnitt ist entsprechend dazu vorgesehen, von dem Trägerfluid im Sterilisationsbetrieb durchströmt zu werden, sodass dann auch ein Durchströmen des Sterilisationsmittelreservoirs ermöglicht wird. Zugleich kann über diesen Kanalabschnitt auch das Behandlungsfluid während des Produktionsbetriebes strömen, wobei dann aber sicherzustellen ist, dass das Sterilisationsmittelreservoir fluiddicht von dem Kanalabschnitt getrennt ist.

Zum Wechsel und/oder zum Befüllen des zumindest einen Sterilisationsmittelreservoirs kann der Kanalabschnitt über ein öffenbaren Wandabschnitt der Sterilisationsvorrichtung zugänglich sein. Entsprechend wird dieser Wandabschnitt geöffnet, wobei dann von außen das Sterilisationsmittelreservoir entnommen oder befüllt werden kann. Zugleich ist bei diesem Schritt allerdings auch sicherzustellen, dass dies unter möglichst kontrollierten Verhältnissen erfolgt, da ein Öffnen der Zuführleitung bzw. der Sterilisationsvorrichtung nach einem Sterilisationsbetrieb bedeutet, dass erneut ein gewisser Keim- oder Bakterieneintrag erfolgen kann. Bevorzugt kann es daher zweckmäßig sein, eine entsprechende Handhabungsvorrichtung innerhalb eines kontrollierten Raums zum Beispiel eines Reinraums vorzusehen, welche automatisiert das Sterilisationsmittelreservoir wechselt bzw. befüllt, sodass für den Anwender keine entsprechenden manuellen Maßnahmen erforderlich sind.

Insbesondere wenn das Sterilisationsmittelreservoir als Sterilisationsmittelpatrone ausgebildet ist, kann auch eine Ausgestaltung von Vorteil sein, bei der die Sterilisationsvorrichtung ein stellbares Magazin zur Aufnahme einer Vielzahl von Sterilisationsmittelreservoiren bzw. Sterilisationsmittelpatronen aufweist, wobei durch Stellung des Magazins zumindest ein Sterilisationsmittelreservoir in den Kanalabschnitt angeordnet werden kann. Entsprechend kann von außen in einfacher Art und Weise ein befülltes Sterilisationsmittelreservoir in dem Magazin angeordnet werden, welches dann durch Verstellung in den Kanalabschnitt befördert wird. Insbesondere kann das Magazin nach Art eines Revolvermagazins ausgebildet sein, wobei stets nach dem Sterilisationsbetrieb das verbrauchte Sterilisationsmittelreservoir aus dem Kanalabschnitt herausgenommen und durch ein neues, befülltes Sterilisationsmittelreservoir ersetzt wird. Gemäß einer Weiterbildung können sich auch die Magazinplätze für Sterilisationsmittelreservoirs abwechseln mit freien Magazinplätzen, wobei diese freien Magazinplätze im Wesentlichen während des Produktionsbetriebes in dem Kanalabschnitt angeordnet sind und wobei erst für den Sterilisationsbetrieb ein Sterilisationsmittelreservoir in den Kanalabschnitt eingebracht wird. Anstelle eines Revolvermagazins ist auch ein einfaches Schiebemagazin denkbar, welches durch Verschieben das entsprechende Sterilisationsmittelreservoir in den Kanalabschnitt einbringt oder herausschiebt.

Gemäß einer bevorzugten Weiterbildung kann das zumindest eine Sterilisationsmittelreservoir und/oder die Sterilisationsvorrichtung eine Heizvorrichtung zum Aufheizen des Sterilisationsmittels aufweisen. Die Aufheizung erfolgt hierbei insbesondere über eine elektrische Heizvorrichtung. Alternativ ist auch eine chemische Heizvorrichtung denkbar, welche allein durch chemische Reaktion eine ausreichende Wärme zum Aufheizen des Sterilisationsmittels ermöglicht. In diesem Zusammenhang sind dann entsprechende Heizelemente oder Reaktionsmittel in der Sterilisationsvorrichtung und/oder dem Sterilisationsmittelreservoir angeordnet. Mithilfe der Heizvorrichtung ist es möglich, das Sterilisationsmittel auf eine Temperatur zu heizen, in der eine Verdampfung erfolgt. Hierdurch kann das Sterilisationsmittel besonders gut von dem Trägerfluid aufgenommen werden. Gerade wenn es sich bei dem Trägerfluid um trockene Luft handelt, ist eine Aufnahme dann in einfacher Art und Weise möglich. Im Falle einer elektrischen Heizvorrichtung sind entsprechende elektrische Kontaktierungen an der Zuführleitung vorgesehen, welche mit dem Sterilisationsmittelreservoir und/oder der Sterilisationsvorrichtung während des Sterilisationsbetriebes in Kontakt stehen.

Besonders geeignet ist in diesem Zusammenhang eine Ausgestaltung, bei der die Heizvorrichtung in Strömungsrichtung vor dem Sterilisationsmittel und/oder vor dem Sterilisationsmittelreservoir zur Aufheizung des Trägerfluids angeordnet ist. Das Sterilisationsmittel wird dann in das aufgeheizte Trägerfluid eingespritzt und heizt sich ebenfalls auf, bis es verdampft. In diesem Fall erfolgt also eine indirekte Aufheizung des Sterilisationsmittels über die Heizvorrichtung. Die Heizvorrichtung ist dann bevorzugt in der Sterilisationsvorrichtung gebildet, wobei die Heizvorrichtung einen Bypasskanal aufweist, welcher von dem Trägerfluid durchströmt wird. Die Heizvorrichtung kann dann leidglich im Sterilisationsbetrieb zugeschaltet werden und/oder der Bypasskanal lediglich im Sterilisationsbetrieb durchströmt werden.

Eine bevorzugte Weiterbildung der Erfindung sieht ferner vor, dass die Zuführleitung stromaufwärts des zumindest einen Sterilisationsmittelreservoirs im Sterilisationsbetrieb mit einer Trägerfluidzufuhr verbindbar ist. In diesem Zusammenhang bezieht sich eine Anordnung stromaufwärts auf den bestimmungsgemäßen Betrieb der Behälterbehandlungsanlage. So erfolgt üblicherweise ausgehend von der Zuführleitung eine Strömung des Blasfluids oder des Trägerfluid in Richtung der Fluidzuführungen. Zum Einbringen des Trägerfluids kann dann eine Trägerfluidzufuhr z. B. in Form einer Anschlussleitung vorgesehen sein, welche mit der Zuführleitung verbindbar ist. Vorzugsweise ist zwischen der Zuführleitung und der Trägerfluidzufuhr ein Ventil angeordnet, welches für den Sterilisationsbetrieb geöffnet und für den Produktionsbetrieb geschlossen werden kann. Die Trägerfluidzufuhr kann dann entsprechend an ein Trägerfluidreservoir oder aber auch an eine Pumpe angeschlossen werden, welche das erforderliche Trägerfluid bereitstellt. Üblicherweise schließt neben der Trägerfluidzufuhr auch eine Behandlungsfluidzufuhr an die Zuführleitung an. Auch diese Behandlungsfluidzufuhr steht bevorzugt über ein Ventil mit der Zuführleitung in einem Produktionsbetrieb in Verbindung, wobei entsprechend dann das Ventil geöffnet wird und während des Sterilisationsbetriebes geschlossen ist. Zugleich ist es auch möglich, ein Wechselventil vorzusehen, welches wahlweise die Zuführleitung mit der Trägerfluidzufuhr oder der Behandlungsfluidzufuhr verbindet.

Eine Weiterbildung der Erfindung sieht ferner vor, dass zwischen dem zumindest einen Sterilisationsmittelreservoir und der Zuführleitung eine Filtervorrichtung angeordnet ist. Je nach Ausgestaltung der Behälterbehandlungsanlage ist es erforderlich, dass auch während des Produktionsbetriebes sichergestellt wird, dass kein keim- oder bakterienbelastetes Behandlungsfluid über die Fluidzuführungen in die Behälter gelangt. Entsprechend ist eine Filtervorrichtung vorgesehen, deren Filterelement bzw. Filterelemente etwaige Keime oder Bakterien herausfiltern. Die Filterelemente können beispielsweise als Aktivkohlefilter, Sterilfilter oder als Partikelfilter ausgebildet sein. Während des Betriebes werden die Filterelemente kontinuierlich kontaminiert, sodass entsprechend nicht nur eine Sterilisation der Zuführleitung sowie der Fluidzuführungen sondern auch eine Sterilisation und Rekonditionierung der Filterelemente erforderlich ist. Auch dies erfolgt üblicherweise durch Durchströmen der Filterelemente mit einem Sterilisationsfluid. Durch die Einbindung des Sterilisationsmittelreservoirs ist es möglich, stromaufwärts der Filtervorrichtung das Sterilisationsfluid zu erzeugen, sodass dann auch die Filtervorrichtung von diesem Sterilisationsfluid durchströmt und im Zuge dessen rekonditioniert wird.

Gemäß einer bevorzugten Weiterbildung der Erfindung ist die Behälterbehandlungsanlage als Blasanlage oder Streckblasanlage ausgebildet. Entsprechend sind dann die Behandlungsmodule als Blasmodule oder Streckblasmodule ausgebildet, wobei die Zufuhrleitungen dazu eingerichtet sind, in einem Produktionsbetrieb ein Blasfluid in einen Behälter einzubringen, welcher in einer als Blasform ausgebildeten Behälteraufnahme angeordnet ist. Bei dem Blasfluid handelt es sich insbesondere um Druckluft.

Gegenstand der Erfindung ist ferner ein Verfahren zum Betreiben einer erfindungsgemäßen Behälterbehandlungsanlage gemäß Patentanspruch 12, wobei in einem Sterilisationsbetrieb ein Trägerfluid in die Zuführleitung eingeleitet wird und das zumindest eine Sterilisationsmittelreservoir zumindest teilweise, insbesondere vollständig, umströmt, wobei im Zuge dessen das Trägerfluid ein in dem Sterilisationsmittelreservoir angeordnetes Sterilisationsmittel aufnimmt und hierdurch ein Sterilisationsfluid bildet, welches anschließend die Zuführleitung des zumindest einen Behandlungsmoduls durchströmt und sterilisiert.

Bei dem Sterilisationsmittel handelt es sich insbesondere um Wasserstoffperoxid (H₂O₂) oder um Peressigsäure, wobei das Sterilisationsmittel mit einer Konzentration von 2 und 50 g/m³ in dem Sterilisationsfluid vorliegt.

Gemäß einer bevorzugten Weiterbildung wird das Sterilisationsmittel vor und/oder während der Durchströmung des Sterilisationsmittelreservoirs erwärmt und infolgedessen während des Sterilisationsmittelbetriebs kontinuierlich verdampft. Die Temperatur richtet sich insbesondere nach Art des verwendeten Sterilisationsmittels, wobei aber zumindest eine Temperatur von mehr als 90 °C vorgesehen ist.

Bei dem Trägerfluid handelt es sich vorzugsweise um Luft, insbesondere um trockene Luft. Das Trägerfluid weist entsprechend einen Wasserdampfgehalt von weniger als 5 % bei einer Temperatur von 90 °C auf.

Eine bevorzugte Weiterbildung des Verfahrens sieht ferner vor, dass nach dem Sterilisationsbetrieb das zumindest eine Sterilisationsmittelreservoir ausgetauscht oder mit Sterilisationsmittel aufgefüllt wird und in einen Produktionsbetrieb gewechselt wird. Alternativ kann der Austausch und die Befüllung auch erst während eines neuen Sterilisationsbetriebes erfolgen. Während des Produktionsbetriebes wird ein Behandlungsfluid, insbesondere ein Blasfluid, in die Zuführleitung eingeleitet, wobei das zumindest eine Sterilisationsmittelreservoir fluiddicht von der Zuführleitung und der Fluidzuführung getrennt ist. Alternativ ist es auch möglich, während des Produktionsbetriebs das Sterilisationsmittelreservoir vollständig zu entnehmen, sodass bereits hierdurch eine fluiddichte Trennung bewirkt werden kann.

Im Folgenden wird die Erfindung anhand exemplarischer Zeichnungen näher erläutert. Es zeigen:
- Fig. 1: eine seine schematische Darstellung der erfindungsgemäßen Behälterbehandlungsanlage,
- Fig. 2: eine Detaildarstellung im Bereich der Sterilisationsvorrichtung mit öffenbaren Wandabschnitt zum Wechsel des Sterilisationsmittelreservoirs,
- Fig. 3: eine alternative Ausgestaltung der Filtervorrichtung mit Magazin.

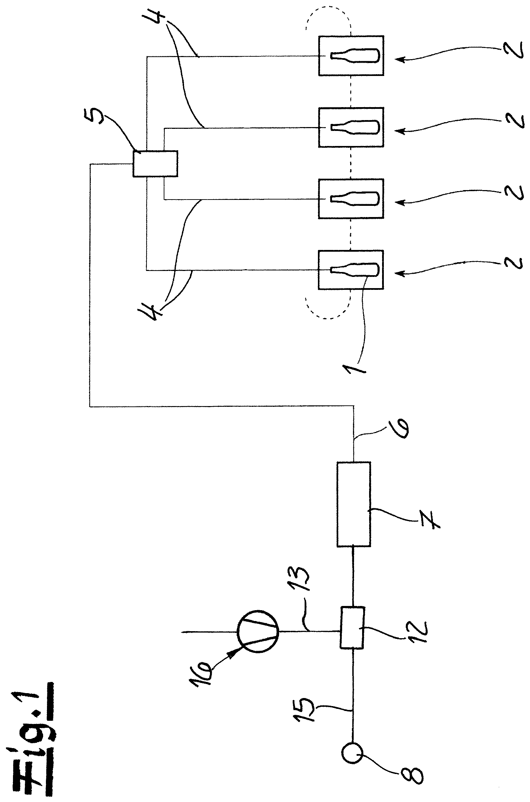

Die Fig. 1 zeigt eine erfindungsgemäße Behälterbehandlungsanlage, welche in dem gezeigten Beispiel als Streckblasmaschine ausgebildet ist und wobei während eines Produktionsbetriebes durch Einbringen eines als Blasfluid ausgebildeten Behandlungsfluids 9 Behälter 1 aus Kunststoffvorformlingen zu Kunststoffflaschen geformt werden. Hierzu sind die Behälter 1 in Behandlungsmodulen 2 angeordnet, welche jeweils eine Fluidzuführung 4 aufweisen, welche über eine Drehdurchführung 5 mit einer Zuführleitung 6 in Verbindung stehen. Zusätzlich ist eine Filtervorrichtung 7 in der Zuführleitung 6 angeordnet, welche während des Produktionsbetriebes das Behandlungsfluid 9 bzw. das Blasfluid filtert, sodass keine Keime oder Bakterien in die Behälter 1 gelangen können.

Die Bereitstellung des Behandlungsfluids 9 erfolgt über eine Behandlungsfluidzufuhr 8, wobei es sich bei dem Behandlungsfluid 9 üblicherweise um Druckluft handelt.

Um die Filtervorrichtung 7 sowie die Zuführleitung 6 und die Fluidzuführungen 4 möglichst keimfrei halten zu können, ist es erforderlich in gewissen Zeitabschnitten eine Sterilisation dieser Komponenten durchzuführen. Entsprechend wird dann in einen Sterilisationsbetrieb gewechselt, in welchem ein Sterilisationsfluid 11 durch die Zuführleitung 6 und damit auch durch die Filtervorrichtung 7 und die Fluidzuführungen 4 geleitet wird und welche diese Komponenten im Zuge dessen sterilisiert.

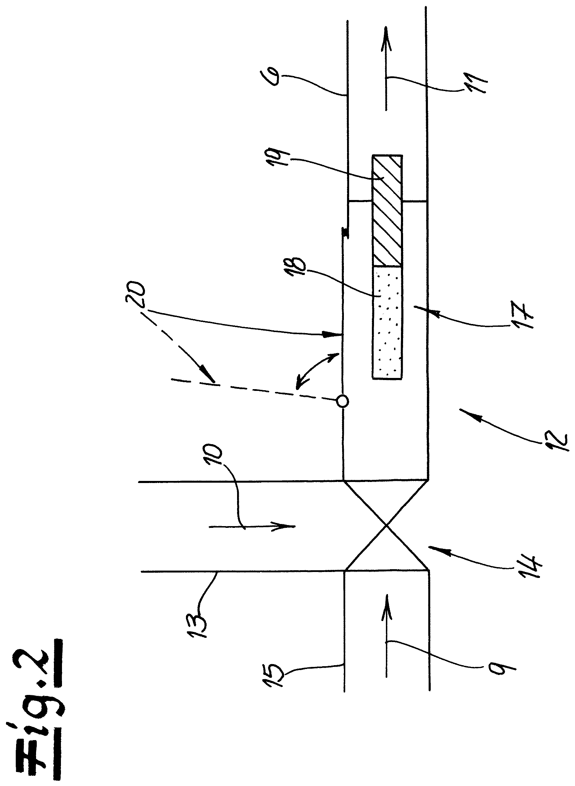

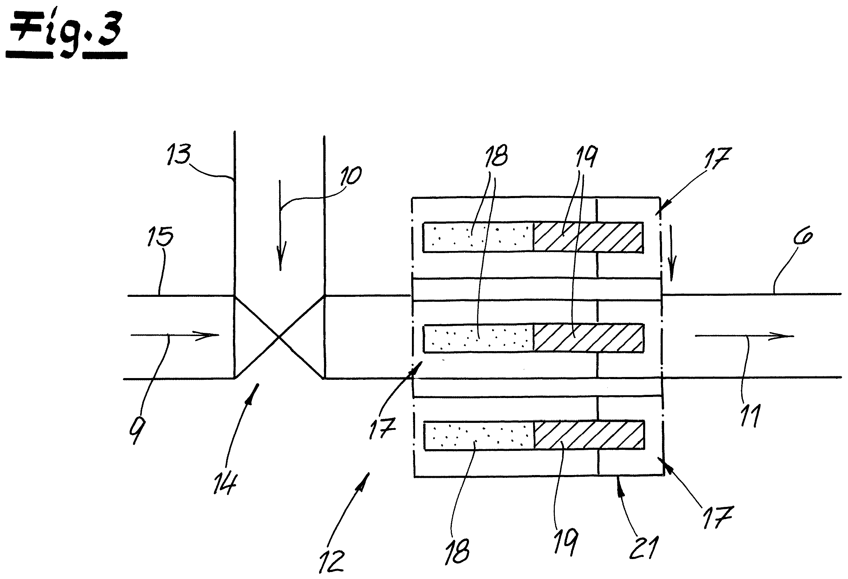

Die Bereitstellung dieses Sterilisationsfluides 11 erfolgt in der Sterilisationsvorrichtung 12, welche in den Fig. 2 und 3 näher erläutert wird.

Anhand der Fig. 2 wird deutlich, dass die Zuführleitung 6 bereichsweise als Sterilisationsvorrichtung 12 ausgebildet ist. Hierzu schließt eine Trägerfluid-führende Leitung 13 über ein Wechselventil 14 an die Zuführleitung 6 an und ermöglicht das Zuführen von einem Trägerfluid 10, wobei es sich vornehmlich um trockene Luft handelt. Während des Produktionsbetriebes kann das Wechselventil 14 dann die Zuführleitung 6 mit einer Behandlungsfluid-führenden Leitung 15 verbunden werden, um entsprechend anstelle des Trägerfluids 10 ein Behandlungsfluid 9 zuzuführen. Die Bereitstellung des Trägerfluids 10 erfolgt über eine in der Fig. 1 dargestellte Druckpumpe 16.

Das Trägerfluid 10 umströmt nunmehr die Sterilisationsvorrichtung 12 und damit zugleich auch ein Sterilisationsmittelreservoir 17 in welchem in einer Kammer Sterilisationsmittel 18 in Form von Wasserstoffperoxid (H₂O₂) angeordnet ist. Darüber hinaus weist das Sterilisationsmittelreservoir 17 auch eine Heizvorrichtung 19 auf, welche in chemischer oder elektronischer Art und Weise ein Aufheizen des Sterilisationsmittels 18 bewirkt und dieses im Zuge dessen verdampft. Das Sterilisationsmittelreservoir 17 ist in dem gezeigten Beispiel als Sterilisationsmittelpatrone ausgebildet und kann von dem Trägerfluid 10 umströmt werden, sodass sich dann aus dem Trägerfluid 10 und dem Sterilisationsmittel 18 das Sterilisationsfluid 11 ergibt.

Das Sterilisationsmittelreservoir 17 ist ferner in einem Kanalabschnitt der Sterilisationsvorrichtung 12 angeordnet, welcher von einem öffenbaren Wandabschnitt 20 begrenzt wird und wobei dieser Wandabschnitt 20 durch die gestrichelte Linie in einem geöffneten Zustand und in der durchgehenden Linie im geschlossenen Zustand dargestellt wird. Durch Öffnen des Wandabschnittes 12 ist es möglich, das Sterilisationsmittelreservoir 17 zu entnehmen und durch ein Neues zu ersetzen. Hierbei ist zu beachten, dass das in dem Sterilisationsmittelreservoir 17 angeordnete Sterilisationsmittel 18 üblicherweise für lediglich einen Sterilisationsvorgang ausreichend ist und sodann erneuert werden muss.

Die Fig. 3 zeigt eine alternative Ausgestaltung, bei der insgesamt drei Sterilisationsmittelreservoirs 17 in einem Magazin 21 angeordnet sind, wobei jedes Sterilisationsmittelreservoir 17 in einem eigenen Magazinplatz angeordnet ist und wobei durch Verstellen der Magazinplätze die einzelnen Sterilisationsmittelreservoirs in den Kanalabschnitt der Sterilisationsvorrichtung eingebracht werden können.

### Bezugszeichenliste

- 1: Behälter
- 2: Behandlungsmodule
- 4: Fluidzuführung
- 5: Drehdurchführung
- 6: Zuführleitung
- 7: Filtervorrichtung
- 8: Behandlungsfluidzufuhr
- 9: Behandlungsfluid
- 10: Trägerfluid
- 11: Sterilisationsfluid
- 12: Sterilisationsvorrichtung
- 13: Trägerfluid-führende Leitung
- 14: Wechselventil
- 15: Behandlungsfluid-führenden Leitung
- 16: Druckpumpe
- 17: Sterilisationsmittelreservoir
- 18: Sterilisationsmittel
- 19: Heizvorrichtung
- 20: Wandabschnitt
- 21: Magazin

## Patentansprüche

1. Behälterbehandlungsanlage zum Behandeln von Behältern (1) mit zumindest einem Behandlungsmodul (2), welches eine Behälteraufnahme sowie eine der Behälteraufnahme zugeordnete Fluidzuführung (4) aufweist, wobei die Fluidzuführung (4) dazu ausgebildet ist, ein Behandlungsfluid (9) in einen Behälter (1) einzubringen und wobei die Fluidzuführung (4) fluidwirkend an eine Zuführleitung (6) anschließt,
**dadurch gekennzeichnet, dass**
in einem Sterilisationsbetrieb zumindest ein Sterilisationsmittelreservoir (17) in der Zuführleitung (6) angeordnet ist, welches fluidwirkend mit der Fluidzuführung (4) des zumindest einen Behandlungsmoduls (2) verbunden ist.

2. Behälterbehandlungsanlage nach Anspruch 1, **dadurch gekennzeichnet, dass** das zumindest eine Sterilisationsmittelreservoir (17) umströmbar ausgebildet ist.

3. Behälterbehandlungsanlage nach einem der vorangegangenen Ansprüche, **dadurch gekennzeichnet, dass** ein Sterilisationsmittel (18), bevorzugt Wasserstoffperoxid, in dem Sterilisationsmittelreservoir (17) angeordnet ist.

4. Behälterbehandlungsanlage nach einem der vorangegangenen Ansprüche, **dadurch gekennzeichnet, dass** das zumindest eine Sterilisationsmittelreservoir (17) als austauschbare Sterilisationsmittelpatrone oder als nachfüllbarer Sterilisationsmitteltank in der Zuführleitung (6) angeordnet ist.

5. Behälterbehandlungsanlage nach Anspruch 4, **dadurch gekennzeichnet, dass** die Zuführleitung (6) abschnittsweise als Sterilisationsvorrichtung (12) ausgebildet ist, wobei das zumindest eine Sterilisationsmittelreservoir (17) in einem fluidwirkend mit der Zuführleitung (6) verbundenen Kanalabschnitt der Sterilisationsvorrichtung (12) angeordnet ist.

6. Behälterbehandlungsanlage nach Anspruch 4, **dadurch gekennzeichnet, dass** der Kanalabschnitt über eine öffenbaren Wandabschnitt (20) der Sterilisationsvorrichtung (12) zum Wechsel und/oder zum Befüllen des zumindest einen Sterilisationsmittelreservoirs (17) zugänglich ist.

7. Behälterbehandlungsanlage nach Anspruch 4 oder 5, **dadurch gekennzeichnet, dass** die Sterilisationsvorrichtung (12) ein stellbares Magazin (21) zur Aufnahme einer Vielzahl von Sterilisationsmittelreservoirs (17) aufweist, wobei durch Stellung des Magazins (21) zumindest ein Sterilisationsmittelreservoir (17) in dem Kanalabschnitt angeordnete werden kann.

8. Behälterbehandlungsanlage nach einem der vorangegangenen Ansprüche, **dadurch gekennzeichnet, dass** das zumindest eine Sterilisationsmittelreservoir (17) und/oder die Sterilisationsvorrichtung (12) eine Heizvorrichtung (19) zum Aufheizen des Sterilisationsmittels (18) aufweisen.

9. Behälterbehandlungsanlage nach einem der vorangegangenen Ansprüche, **dadurch gekennzeichnet, dass** die Zuführleitung (6) stromaufwärts des zumindest einen Sterilisationsmittelreservoirs (17) im Sterilisationsbetrieb mit einer Trägerfluidzufuhr (11) verbindbar ist.

10. Behälterbehandlungsanlage nach einem der vorangegangenen Ansprüche, **dadurch gekennzeichnet, dass** zwischen dem zumindest einen Sterilisationsmittelreservoir (12) und der Zuführleitung (6) eine Filtervorrichtung (7) angeordnet ist.

11. Behälterbehandlungsanlage nach einem der vorangegangenen Ansprüche, **dadurch gekennzeichnet, dass** die Behandlungsmodule (2) als Blasmodule oder Streckblasmodule ausgebildet sind, wobei die Zuführleitungen (6) dazu eingerichtet sind, in einem Produktionsbetrieb ein Blasfluid in einen Behälter (1) einzubringen, welcher in einer als Blasform ausgebildeten Behälteraufnahme angeordnet ist.

12. Verfahren zum Betreiben einer Behälterbehandlungsanlage nach einem der vorangegangenen Ansprüche, **dadurch gekennzeichnet, dass** in einem Sterilisationsbetrieb ein Trägerfluid (10) in die Zuführleitung (6) eingeleitet wird und das zumindest eine Sterilisationsmittelreservoir (17) umströmt, wobei im Zuge dessen das Trägerfluid (10) ein in dem Sterilisationsmittelreservoir (17) angeordnetes Sterilisationsmittel (18) aufnimmt und ein Sterilisationsfluid (11) bildet, welches anschließend die Zuführleitung (6) des zumindest einen Behandlungsmoduls (2) umströmt und sterilisiert.

13. Verfahren nach Anspruch 12, **dadurch gekennzeichnet, dass** das Sterilisationsmittel (18) vor und/oder während der Umströmung des Sterilisationsmittelreservoirs (17) erwärmt wird und in Folge dessen während des Sterilisationsbetriebs kontinuierlich verdampft.

14. Verfahren nach Anspruch 13, **dadurch gekennzeichnet, dass** das Sterilisationsmittel (18) auf eine Temperatur größer als 90 °C erwärmt wird.

15. Verfahren nach einem der Ansprüche 12 bis 14, **dadurch gekennzeichnet, dass** als Trägerfluid (10) Luft, insbesondere trockene Luft, in die Zuführleitung (6) eingeleitet wird.

16. Verfahren nach einem der Ansprüche 12 bis 15, **dadurch gekennzeichnet, dass** nach dem Sterilisationsbetrieb das zumindest eine Sterilisationsmittelreservoir (17) ausgetauscht oder mit Sterilisationsmittel (18) aufgefüllt wird und in einen Produktionsbetrieb gewechselt wird, wobei ein Behandlungsfluid (9), insbesondere ein Blasfluid, in die Zuführleitung (6) eingeleitet wird und wobei das zumindest eine Sterilisationsmittelreservoir (17) fluiddicht von der Zuführleitung (6) und der Fluidzuführung (4) getrennt ist.

## Claims

1. A container treatment system for treating containers (1) with at least one treatment module (2), which has a container receptacle and a fluid feed (4) assigned to the container receptacle, wherein the fluid feed (4) is designed to introduce a treatment fluid (9) into a container (1), and wherein the fluid feed (4) fluid-actively adjoins a feed line (6), **characterised in that** in a sterilisation mode, at least one sterilising agent reservoir (17) is arranged in the feed line (6) and is connected fluid-actively to the fluid feed (4) of the at least one treatment module (2).

2. The container treatment system according to Claim 1, **characterised in that** the at least one sterilising agent reservoir (17) is designed to allow flow to pass around it.

3. The container treatment system according to any one of the preceding claims, **characterised in that** a sterilising agent (18), preferably hydrogen peroxide, is arranged in the sterilising agent reservoir (17).

4. The container treatment system according to any one of the preceding claims, **characterised in that** the at least one sterilising agent reservoir (17) is arranged as an exchangeable sterilising agent cartridge or as a refillable sterilising agent tank in the feed line (6).

5. The container treatment system according to Claim 4, **characterised in that** the feed line (6) is designed in some portions as a sterilising device (12), wherein the at least one sterilising agent reservoir (17) is arranged in a duct portion, connected fluid-actively to the feed line (6), of the sterilising device (12).

6. The container treatment system according to Claim 4, **characterised in that** the duct portion is accessible via an openable wall portion (20) of the sterilising device (12) in order to change and/or fill the at least one sterilising agent reservoir (17).

7. The container treatment system according to Claim 4 or 5, **characterised in that** the sterilising device (12) has a positionable magazine (21) for accommodating a plurality of sterilising agent reservoirs (17), wherein at least one sterilising agent reservoir (17) can be arranged in the duct portion by positioning the magazine (21).

8. The container treatment system according to any one of the preceding claims, **characterised in that** the at least one sterilising agent reservoir (17) and/or the sterilising device (12) have a heating device (19) for heating the sterilising agent (18).

9. The container treatment system according to any one of the preceding claims, **characterised in that** the feed line (6) can be connected to a carrier fluid feed (11) upstream of the at least one sterilising agent reservoir (17) during sterilisation mode.

10. The container treatment system according to any one of the preceding claims, **characterised in that** a filter device (7) is arranged between the at least one sterilising agent reservoir (12) and the feed line (6).

11. The container treatment system according to any one of the preceding claims, **characterised in that** the treatment modules (2) are designed as blowing modules or stretch blow modules, wherein the feed lines (6) are configured, in a production mode, to introduce a blowing fluid into a container (1) that is arranged in a container receptacle designed as a blowing mould.

12. A method for operating a container treatment system according to any one of the preceding claims, **characterised in that**, in a sterilisation mode, a carrier fluid (10) is conducted into the feed line (6) and flows around the at least one sterilising agent reservoir (17), wherein, in the course of this, the carrier fluid (10) picks up a sterilising agent (18) arranged in the sterilising agent reservoir (17) and forms a sterilising fluid (11), which then flows around and sterilises the feed line (6) of the at least one treatment module (2).

13. The method according to Claim 12, **characterised in that** the sterilising agent (18) is heated before and/or while flow passes around the sterilising agent reservoir (17) and as a result is continuously evaporated during sterilisation mode.

14. The method according to Claim 13, **characterised in that** the sterilising agent (18) is heated to a temperature greater than 90°C.

15. The method according to any one of Claims 12 to 14, **characterised in that** air, in particular dry air, is conducted as the carrier fluid (10) into the feed line (6).

16. The method according to any one of Claims 12 to 15, **characterised in that**, after sterilisation mode, the at least one sterilising agent reservoir (17) is exchanged or filled with sterilising agent (18) and switched to a production mode, wherein a treatment fluid (9), in particular a blowing fluid, is conducted into the feed line (6), and wherein the at least one sterilising agent reservoir (17) is isolated fluid-tightly from the feed line (6) and the fluid feed (4).

## Revendications

1. Installation de traitement de contenants, destinée à traiter des contenants (1), pourvue d'au moins un module de traitement (2), lequel comporte un réceptacle de contenants, ainsi qu'une alimentation en fluide (4), associée au réceptacle de contenants, l'alimentation en fluide (4) étant conçue pour introduire un fluide de traitement (9) dans un contenant (1) et l'alimentation en fluide (4) se raccordant en communication fluidique sur une conduite d'alimentation (6), **caractérisée en ce que** dans un mode de stérilisation, au moins un réservoir (17) d'agent stérilisant, lequel est en liaison en communication fluidique avec l'alimentation en fluide (4) de l'au moins un module de traitement (2) est placé dans la conduite d'alimentation (6).

2. Installation de traitement de contenants selon la revendication 1, **caractérisée en ce que** l'au moins un réservoir (17) d'agent stérilisant est conçu pour être contourné par un écoulement.

3. Installation de traitement de contenants selon l'une quelconque des revendications précédentes, **caractérisée en ce qu'**un agent stérilisant (18), de manière préférentielle un peroxyde d'hydrogène est placé dans le réservoir (17) d'agent stérilisant.

4. Installation de traitement de contenants selon l'une quelconque des revendications précédentes, **caractérisée en ce que** l'au moins un réservoir (17) d'agent stérilisant est placé sous la forme d'une cartouche d'agent stérilisant interchangeable ou d'une cuve d'agent stérilisant rechargeable dans la conduite d'alimentation (6).

5. Installation de traitement de contenants selon la revendication 4, **caractérisée en ce que** la conduite d'alimentation (6) est conçue par tronçons sous la forme d'un dispositif de stérilisation (12), l'au moins un réservoir (17) d'agent stérilisant étant placé dans un tronçon de canalisation du dispositif de stérilisation (12) relié en communication fluidique avec la conduite d'alimentation (6).

6. Installation de traitement de contenants selon la revendication 4, **caractérisée en ce que** le tronçon de canalisation est accessible par un tronçon de paroi (20) pouvant s'ouvrir du dispositif de stérilisation (12), pour remplacer / et ou pour remplir l'au moins réservoir (17) d'agent stérilisant.

7. Installation de traitement de contenants selon la revendication 4 ou 5, **caractérisée en ce que** le dispositif de stérilisation (12) comporte un magasin (21) ajustable, destiné à recevoir une pluralité de réservoirs (17) d'agent stérilisant, par ajustage du magasin (21), au moins un réservoir (17) d'agent stérilisant pouvant être placé dans le tronçon de canalisation.

8. Installation de traitement de contenants selon l'une quelconque des revendications précédentes, **caractérisée en ce que** l'au moins un réservoir (17) d'agent stérilisant et / ou le dispositif de stérilisation (12) comportent un dispositif de chauffage (19), destiné à chauffer l'agent stérilisant (18).

9. Installation de traitement de contenants selon l'une quelconque des revendications précédentes, **caractérisée en ce qu'**en mode de stérilisation, en amont de l'au moins un réservoir (17) d'agent stérilisant, la conduite d'alimentation (6) peut se relier avec une alimentation (11) de fluide porteur.

10. Installation de traitement de contenants selon l'une quelconque des revendications précédentes, **caractérisée en ce qu'**entre l'au moins un réservoir (12) d'agent stérilisant et la conduite d'alimentation (6) est placé un dispositif de filtration (7).

11. Installation de traitement de contenants selon l'une quelconque des revendications précédentes, **caractérisée en ce que** les modules de traitement (2) sont conçus sous la forme de modules de soufflage ou de modules d'étirage-soufflage, les conduites d'alimentation (6) étant configurées pour, dans un mode de production introduire un fluide de soufflage dans un contenant (1), lequel est placé dans un réceptacle de contenants conçu sous la forme d'un moule de soufflage.

12. Procédé opérationnel d'une installation de traitement de contenants selon l'une quelconque des revendications précédentes, **caractérisé en ce que** dans un mode de stérilisation, un fluide porteur (10) est injecté dans la conduite d'alimentation (6) et contourne par écoulement l'au moins un réservoir (17) d'agent stérilisant, à cette occasion, le fluide porteur (10) absorbant un agent stérilisant (18) placé dans le réservoir (17) d'agent stérilisant et constituant un fluide de stérilisation (11), lequel contourne ensuite par écoulement et stérilise la conduite d'alimentation (6) de l'au moins un module de traitement (2).

13. Procédé selon la revendication 12, **caractérisé en ce que** l'agent stérilisant (18) est chauffé avant et / ou pendant le contournement par écoulement du réservoir (17) d'agent stérilisant et en conséquence de quoi, s'évapore en continu pendant le mode de stérilisation.

14. Procédé selon la revendication 13, **caractérisé en ce que** l'agent stérilisant (18) est chauffé à une température supérieure à 90 °C.

15. Procédé selon l'une quelconque des revendications 12 à 14, **caractérisé en ce que** de l'air, notamment de l'air sec est injecté en tant que fluide porteur (10) dans la conduite d'alimentation (6).

16. Procédé selon l'une quelconque des revendications 12 à 15, **caractérisé en ce qu'**après le mode de stérilisation, l'au moins un réservoir (17) d'agent stérilisant est changé ou rempli d'agent stérilisant (18) et remplacé dans un mode de production, un fluide de traitement (9), notamment un fluide de soufflage étant injecté dans la conduite d'alimentation (6) et l'au moins un réservoir (17) d'agent stérilisant étant séparé de manière étanche au fluide de la conduite d'alimentation (6) et de l'alimentation en fluide (4).
